# EUROPEAN PATENT SPECIFICATION

(11) **EP 2 670 714 B1**
(45) Date of publication and mention of the grant of the patent: **26.07.2017**
(21) Application number: 12742063.6
(22) Date of filing: 02.02.2012
(51) Int. Cl.: C02F 3/00, C10G 32/00, C22B 3/18, B09C 1/10, C12C 11/07, C05F 17/00, C12M 1/38, C12M 1/34, C22B 3/02, C02F 101/00, C02F 103/00, C02F 103/06, C02F 103/10

(54) **APPARATUS AND METHOD FOR CONDUCTING MICROBIOLOGICAL PROCESSES**
VORRICHTUNG UND VERFAHREN ZUR DURCHFÜHRUNG MIKROBIOLOGISCHER PROZESSE
APPAREIL ET MÉTHODE POUR RÉALISER DES PROCESSUS MICROBIOLOGIQUES

(30) Priority: 02.02.2011 ZA 201100857
(43) Date of publication of application: 11.12.2013
(73) Proprietor: University Of The Free State, Bloemfontein 9301 (ZA)
(72) Inventor: ERASMUS, Johan, 6525 South Africa (ZA); VAN HEERDEN, Estariethe, Bloemfontein 9301 (ZA)
(74) Representative: Potter Clarkson LLP
(86) International application number: PCT/IB2012/000173
(87) International publication number: WO 2012/104717

(56) References cited:
- EP-A1- 0 461 144
- WO-A1-00/71763
- DE-A1- 10 024 547
- US-A- 3 933 628
- US-A- 4 849 360
- US-A- 5 362 397
- US-A- 5 478 464
- US-A1- 2004 191 755
- US-B1- 6 428 694

## Description

### FIELD OF THE INVENTION

This invention relates to an energy efficient apparatus and method for conducting microbiological processes on bulk materials such as soil, sand, granular ores, water, sub-divided biodegradable waste that is to be biologically treated, as well as possibly other subdivided bulk materials capable of microbiological processing.

The invention is particularly, although not exclusively, concerned with microbiological processes that are energy sufficient, in particular by utilizing renewable energy in self sustaining low cost cells, wherein biological processes are used to treat bulk materials in order to decontaminate or biotransform them to environmentally more friendly products or to extract components from them. The biological processes preferably involve the use of naturally occurring microbiota/biome to effect desired biological activity within the bulk material. The microbial communities may include bacteria archaea, eucarya and even viral biomes.

In one important application, the invention is directed at the bioremediation of contaminated soil, water or sand, such as that contaminated with spilt petroleum products such as petrol or gasoline, aviation fuel, diesel fuel or other exogenous contaminants.

In many aspects of the invention its use enables energy efficient microbiological processes to be carried out near the locality in which the bulk material is present in a highly effective manner with a result that the carbon footprint of certain situations is improved by using renewable energy.

### BACKGROUND TO THE INVENTION

It is well known that many beneficial microbiological processes, especially bacterial processes, take place naturally and each different process involves the activity of different species/genera of bacteria. The speed of catalysis in the relevant bacteria is, however, also dependent on prevailing physicochemical conditions especially as regards the presence of moisture and oxygen in the bulk material being treated and the temperature.

Numerous different microbiological processes have accordingly been proposed in which at least some control of the ambient conditions is exercised with a view to accelerating the microbiological activity.

Furthermore, the use of so-called "BIO-cells" has been proposed for the bioremediation of fuel/hydrocabon contaminated soils on site and in which oxygen is supplied in the form of air.

There are numerous different human endeavors that result in contamination such as in the mining, industrial, and agricultural fields and each generally produces associated waste that requires disposal.

Any site that has contamination is morally if not legally obliged to select from a wide array of treatment options with efficacy and cost being major factors in making a decision. Many countries rarely consider *in situ* or on site approaches although, with bulk materials such as soil, they would often be less costly and can be done in a shorter time frame and pose less risk.

In this regard the US Navy's TechData Sheet TDS-2017-ENV (2nd Revision) describes bio cells in which the addition of moisture and nutrients such as nitrogen and phosphorus can be used to enhance microbial activity and wherein provision is made for the removal of leachate from soil being processed in a large container. The bio cell also provides for the extraction of volatile organic compounds released by passing the off-gases through a granulated activated carbon adsorption system. Whilst providing an effective bioremediation expedient, the bio cells described in this publication nevertheless consume energy and thus have associated with them a considerable operating cost. Also, these bio cells operate at ambient temperature and the microbiological activity is associated with the prevailing temperature. This is so to the extent that in certain climates in which the temperature decreases substantially in winter months, bioremediation sites need to be closed for the coldest part of the year.

It is to be noted that whilst the better known microbial processes for the degradation of hydrocarbons are aerobic, it is common cause that there are many anaerobic and even anoxic microbes that can effectively bio remediate soils as well as extract valuable components from subdivided ores or the like.

A need is perceived for a method of conducting a microbiological process on bulk materials in which the microbiological process is carried out under conditions that enhance microbiological activity, and therefore, as a general rule, reduce the time taken for a microbiological process to achieve a predetermined result.

Such a bio cell for the conduct of microbiological processes on bulk materials of the general nature outlined above should be satisfactorily cost effective. Such a bio cell in application to bulk materials would preferably, although not necessarily, utilize *in situ* communities and metabolic functionality of microbiological species. Such a bio cell would preferably be relatively easy to move from one site to another.

It is also preferable that a method and apparatus for conducting a microbiological process be one in which the conduct of the process is aimed at reducing the ecological footprint of at least particular situations, especially, although not exclusively, in the bioremediation field.

A method and apparatus for conducting such a microbiological process will preferably use offsite control or PLC control utilizing feedback data to adjust feed or physicochemical parameters to enhance bioremediation or bio activity.

US5362397 describes a method for the biodegradation of organic contaminants in a mass of particulate solids. The method comprises providing a contaminated mass of particulate solids on an impervious surface in fluid communication with an impervious recovery reservoir. The impervious surface has operating thereon air supply and/or air suction means to provide suitable and continuous oxygenation of the mass and/or to remove undesirable vapour emissions from the mass. The mass is periodically irrigated by applying on its surface a culture medium comprising at least one bacterial strain and co-substances thereof. The air supplied to the pass of particulate solids is moved by an external compressor and heated or cooled by an external air conditioner.

EP0461144 describes a process for the microbiological remediation of soil polluted by chlorophenols.

### SUMMARY OF THE INVENTION

In accordance with a first aspect of the invention there is provided a method of conducting a microbiological process on a bulk material in which a quantity of the bulk material is loaded onto a waterproof lining forming part of a bio cell with a heat transfer arrangement below the quantity of bulk material or within its volume, or both, and wherein the moisture content of the bulk material is controlled by periodic or intermittent distribution of water into the bulk material in order to promote microbiological activity within the bulk material by means of microbes that may be either naturally occurring within the bulk material or may be selected and introduced into the bulk material according to a desired result, and a leachate recovery installation for collecting leachate draining from the bulk material, in use, wherein the temperature within the bulk material is monitored and the heat transfer arrangement is heated or cooled, as may be required, in order to control the temperature thereof to cause the temperature of the bulk material to approach a target temperature associated with enhanced microbial activity of microbes present within the bulk material wherein the heat transfer arrangement operates to heat or cool air that is fed into an air inlet prior to discharge of the air into the bulk material by way of an air inlet arrangement that embodies a heat exchanger whereby air being fed to the air inlet arrangement is heated or cooled according to the temperature of fluid circulated through the heat exchanger from a heat source and wherein water is heated in a solar water heater for circulation through the heat exchanger as and when heating of air introduced into the bulk material is required.

Further features of the first aspect of the invention provide for the microbes to be selected from aerobic, anaerobic and anoxic types; in the event that air is used to be cooled for cooling to be effected using a suitable air-conditioner; for the heat transfer arrangement to include a heat sink composed of a multitude of pebbles or particles having a heat content aimed at maintaining a generally even temperature during periods of time for which a heat source or source of cooling is inactive; and for the heat source or source of cooling to be a renewable energy type of heat source or source of cooling, especially a solar heat absorption facility, and most especially one having a plurality of inclined evacuated heat absorption tubes.

Still further features of the first aspect of the invention provide for nutrients required for the targeted microbial action, typically nitrogen and phosphorous in addition to oxygen contained in the air, to be optionally added in the form of solid material at the time that the quantity of bulk material is loaded into the bio cell; in the alternative, or in addition, for nutrients to be added, as may be required, by way of water distributed into the bulk material; for water to be distributed into the bulk material by spraying it on to the upper surface thereof; for the water to be recycled leachate optionally together with makeup water that may be added to compensate for any losses or to compensate for a bleed stream of leachate that may be removed; for the nutrient content of the leachate to be monitored and with nutrients being added as may be required where leachate is recirculated; and for the moisture content of the bulk material to be monitored with the distribution of water into the bulk material being controlled according to the moisture content detected.

Yet further features of the first aspect of the invention provide for the entire method to be optionally carried out in an enclosed environment, conveniently in a suitable covering tunnel in which the tunnel is formed as an enclosure together with the waterproof lining of the bio cell; for the enclosed environment to have an outlet for gases that may optionally be fitted with an auger or turbine for extracting energy from gases leaving the enclosed environment; for any outlet gases to be passed through an appropriate scrubber for removing any harmful components thereof; and for a retractable insulating cover to be associated with the tunnel for selectively controlling heat loss through the tunnel wall according to external ambient temperature.

In accordance with a second aspect of the invention there is provided apparatus in the form of a bio cell for the conduct of a method as defined above comprising a waterproof lining; a heat transfer arrangement adapted to be covered by a quantity of bulk material, in use; a water inlet arrangement including flow regulator means whereby water can be periodically or intermittently distributed in bulk material supported above the waterproof lining; at least one moisture detector for detecting the moisture content of bulk material within the container; a leachate recovery installation for collecting leachate draining from bulk material supported above the waterproof lining in use; and at least one temperature detector for detecting temperature within bulk material supported above the waterproof lining; wherein the heat transfer arrangement is arranged, as may be required in use, to adjust the temperature of bulk material supported above the waterproof lining wherein the heat transfer arrangement is arranged to heat or cool air that is fed into an air inlet arrangement prior to its discharge into the bulk material, the heat transfer arrangement including a heat exchanger that may be heated or cooled by fluid circulated through the heat exchanger from a suitable source and wherein a solar water heater is included heating water for circulation through the heat exchanger as and when heating of air introduced into bulk material loaded into the apparatus is required.

Further features of the second aspect of the invention provide for the apparatus to include a controller having an electronic micro-processor with the controller having inputs for association with the at least one temperature detector and the at least one moisture detector; for the controller to have an output for controlling the flow of heating or cooling fluid to the heat transfer arrangement according to the temperature detected by the at least one temperature detector; for the controller to have an output for controlling the flow of water to the water inlet arrangement according to the output from the at least one moisture detector; for the apparatus to include nutrient detector means for detecting nutrients in the leachate in which instance the controller has an input for the output from the nutrient detector means and, in the event that the leachate is recycled, for the controller to optionally control the addition of nutrients to water/leachate being supplied to the water inlet arrangement; and for the controller to have associated with it an electrical power supply including a battery unit and a solar photovoltaic cell arrangement for charging the battery unit.

Still further features of the second aspect of the invention provide for the heat exchanger to form a part of the air inlet arrangement with the heat exchanger conveniently receiving heated fluid, in use, from a renewable energy conversion unit, especially a fluid heating solar energy conversion unit that may, in particular, be either a plurality of evacuated solar heat collection tubes or an alternative type of heat collection panel, in either event typically of a type used for heating water; for the heat transfer arrangement to be operatively surrounded by a multitude of pebbles or particles having a heat content aimed at maintaining an elevated temperature during periods of time for which the heat source is inactive and thereby acting as a heat sink; and for the apparatus to include a geo-textile layer for separating the bulk material from the heat sink and air inlet arrangement.

Additional features of the second aspect of the invention provide for the apparatus to include impervious sheet material preferably in the form of a tunnel that fully encloses the bio cell with a covering sheet of material and the lining of the container together acting to form a totally enclosed tunnel for the bio cell with an optional outlet for off-gases in which instance there may be associated with the outlet an auger or turbine for extracting energy from gases leaving the enclosed environment and optionally an appropriate scrubber for removing any harmful components thereof; and for a retractable insulating cover to be associated with the tunnel for selectively controlling heat loss through the tunnel wall according to prevailing external ambient temperature in which instance the controller may be arranged to automatically adjust the position of the retractable insulating, according to ambient temperature fed to the controller by an ambient temperature sensor.

It will be understood that in instances in which added heat is derived from a renewable energy source and electrical energy for operating the controller and any refrigeration or air conditioning apparatus is derived from the same or a different renewable energy source, the entire apparatus becomes a standalone apparatus not needing any other energy input. This being so, at worst, the apparatus provided by the invention would be carbon neutral and, as a general rule, at least in bioremediation applications, the apparatus will, in use, serve to reduce the ecological footprint.

One of the advantages of utilising DC current that is the natural product of solar photovoltaic cells is direct current and the use of batteries to store the electrical energy retains the characteristic of direct current. It is therefore appropriate to utilise direct current motors and pumps for intermittent feeding of water, optionally containing added nutrients, and of air in the case of an aerobic microbe system. It is also noted that energy efficient DC air conditioning units are presently becoming more commonly available and the use of such an air conditioning unit, or a similar refrigeration units may be appropriate for controlling a temperature in areas in which high ambient temperatures are experienced such as in some desert regions where the ambient temperature may arise above an ideal temperature for the growth of the relevant microbes.

A further advantage of utilising DC is that pulsed flow can conveniently be employed whenever it would be advantageous to do so. Pulsed flow can have a variety of different benefits such as the prevention of biological hotspots in the case of a nutrient feed.

In the event that artificial lighting of any type is employed within the bio cell, the lights could be switched in any appropriate manner including short pulsed periods of time. Such lights could, for example, be LEDs of a suitable nature.

The invention may be applied to bioremediation processes such as the remediation of soil, water, heavy metals and sand contaminated with petroleum products, the latter being a particularly important application of the invention.

However, it is envisaged that the invention will also be applied in many other instances such as the bacterial leaching of valuable metals and minerals from ores containing same.

It will be appreciated that the method and apparatus of this invention can be operated remotely by way of cooperating two-way communications devices in which instance an on-site communications device could be employed to transmit current control variables to a remote communications device and the latter could be employed to send back control messages for changing any one or more process variables, as may be required.

Of course, it is also possible to have a one-way communications arrangement in which information as to the status on site can be transmitted to an off-site receiver and a responsible person could take appropriate action by any available means.

In many instances practise of the invention reduces the ecological footprint of at least many different biodegradable waste materials.

In order that the invention may be more fully understood a further more detailed discussion thereof follows with reference to the accompanying drawings.

### BRIEF DESCRIPTION OF THE DRAWINGS

In the drawings:-
- Figure 1: is a schematic system diagram showing the container in partial section and the other components of the apparatus in association therewith;
- Figure 2: is a plan view of the container illustrating the various layers within the container stripped away one by one;
- Figure 3: is a schematic sectional elevation of a part of the length of the heat exchanger of the apparatus; and,
- Figure 4: is a schematic system diagram similar to Figure 1 but showing a system appropriate to the growth of anaerobic or anoxic microbes and further showing another variation of the invention.

### DETAILED DESCRIPTION WITH REFERENCE TO THE DRAWINGS

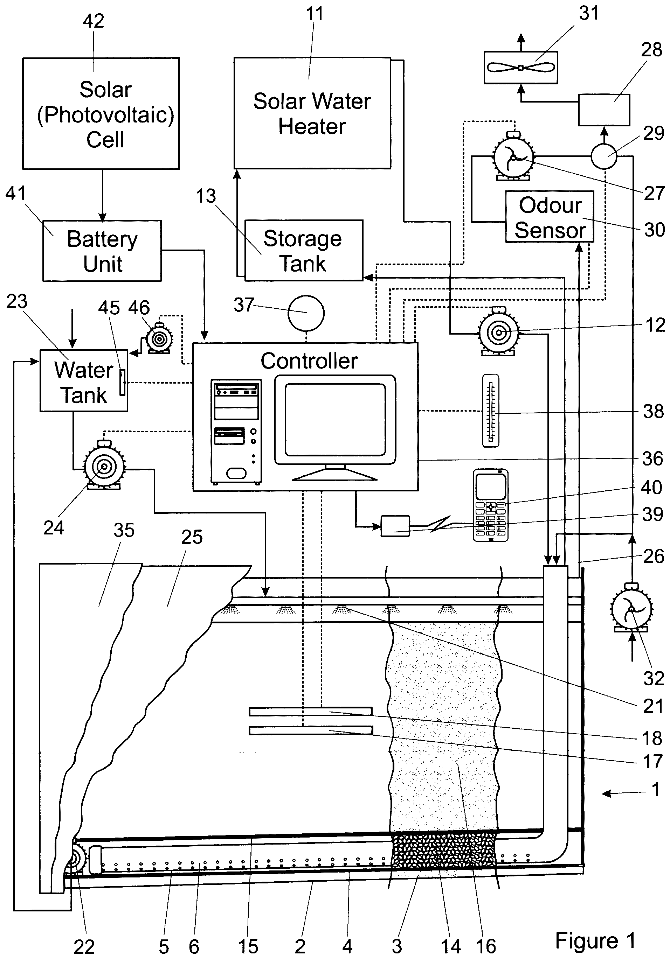

In the embodiment of the invention of which the apparatus is illustrated in Figures 1 to 3 of the drawings, a bio cell for the conduct of a bio remediation process such as that of soil contaminated with petroleum products, comprises a large container (1), typically of a size suitable for containing an appropriate quantity of soil, say from 5 to 20 cubic metres. The container could be a conventional skip of an appropriate size or a suitable shipping container with the top removed, or any other suitable large container that may, of course, even be custom-built for the purpose. Of course, it is also within the scope of this invention that extremely long tunnels that may be generally self-supporting or may be located in temporary or permanent trenches at least partially dug in the Earth's surface could be employed.

Such elongate bio cells could have a length of many metres and up to 100 and even 300 metres, depending on convenience and the environment.

Reverting to the present embodiment of the invention, the bottom (2) of the container is covered with a layer of sand (3) on top of which is placed a waterproof lining (4), typically of a suitable gauge of black polyethylene sheet material. The purpose of the sand is to prevent any hard unevenness on the bottom of the container from perforating the waterproof sheet as the sheet serves the most important purpose of preventing any potentially toxic or harmful liquids from escaping from the bio cell.

On top of the waterproof sheet is a combination air inlet and heat exchanger assembly (5) that includes at least one large diameter plastic distribution pipe (6) having perforations in the lower surface thereof that form an outlet into the container. This arrangement of perforations ensures that any soil or debris does not enter the distribution pipe from the top under the influence of gravity. Depending on the size of the container and the physical arrangement of the various components of the apparatus, there may be more than one such large diameter distribution pipe in which instance it is envisaged that they would generally be arranged in laterally spaced parallel relationship relative to each other.

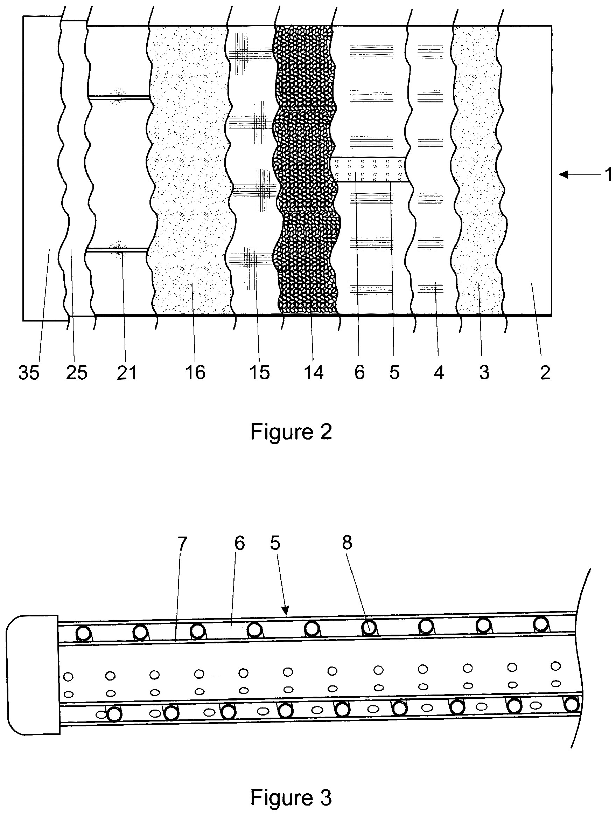

As shown in Figure 3, a smaller diameter air inlet pipe (7), that is also perforated, is located generally concentrically within the distribution pipe. The air inlet pipe is held in spaced relationship relative to the inside surface of the distribution pipe by means of a helically wound heater pipe (8) through which hot water is to be circulated, in use, in order to elevate the temperature of air passing through the air inlet and heat exchanger assembly. Of course coming in the instance that cooling is necessary, cold water could also be circulated in the same way in order to cool air passing through the heat exchanger. The hot water supply to the helically wound heater pipe emanates from a solar water heater (11) by way of a circulation pump (12) that circulates water within a closed circuit including a storage tank (13).

The air supply to the air inlet pipe is described more fully below.

The combination of air inlet and heat exchanger assembly is covered by a permeable body of pebbles (14) that serves to retain heat in the manner of a heat sink so that the temperature within the bio cell does not fluctuate too much with higher and lower day and night temperatures, as will become more apparent from what follows. Also, the permeability of the body of pebbles enables an even distribution of air to be achieved underneath the bulk material being treated, that is contaminated soil in this instance.

Above the body of pebbles is a geotextile layer (15) that in use serves to prevent the bulk material being treated in the bio cell, in this instance the soil that is indicated by numeral (16), from entering the body of pebbles or the combination air inlet and heat exchanger assembly.

It will be understood that the bulk material being treated is introduced into the container within the waterproof lining that extends up the side walls of the container to form an entirely waterproof surround to the bulk material. The bulk material is generally introduced into the container stepwise using an appropriate type of mechanical shovel such as a front end loader. During the loading process, at least one moisture detector (17) for detecting the moisture content of the bulk material within the container is buried at one or more suitable positions within the bulk material. Similarly, at least one temperature detector (18) for detecting temperature at an appropriate position within bulk material in the container is also buried within the bulk material.

Also during the loading process, any solid nutrients, typically in the form of fertilizers containing phosphorus and nitrogen in appropriate proportions, may be added according to requirements of the particular microbiological process that is targeted to take place in the bio cell.

A water inlet arrangement in the form of rows of sprinklers (21) is installed above the upper surface of the bulk material in the container so that water that may contain dissolved nutrients and any other beneficial constituents can be periodically or intermittently distributed onto bulk material contained within the container. In this regard it will be quite apparent to those skilled in the art that the amount of water circulated through the system should not be excessive but should be aimed at maintaining a satisfactory level of moisture within the bulk material that is appropriate to optimum desired microbiological activity.

The water circulation installation includes a leachate pump (22) for pumping leachate draining from bulk material in the container in use and returning it to a water supply tank (23) from which it is pumped by means of a water supply pump (24) to the sprinklers. The leachate pump is activated according to the level of leachate at the bottom of the container within the waterproof lining. It is to be mentioned that at least the waterproof lining, and possibly also the bottom of the container, are preferably inclined so that the leachate pump can be located at a lowermost position in order to re-circulate leachate to the water supply tank.

The entire bio cell is formed into a tunnel (25) comprising an impervious sheet material that cooperates with the lining of the container to fully enclose the bio cell within the cooperating waterproof sheets of material. An outlet that is indicated by numeral (26) is provided for off-gases leaving the enclosed environment. A blower (27) serves to assist in the removal of the off-gases and either recirculating them to the combination air inlet and heat exchanger or discharging them, as may be appropriate, by way of a scrubber (28), such as an activated carbon filter.

A three way valve (29) may be provided to control and optionally divide the flow of off-gases as may be required. The control of the three-way valve may be dependent on the nature of gases detected by an additional sensor (30) that may be of the general type known as an odour sensor. Also, there may be provided an optional energy recovery device such as an auger or turbine type of rotary device (31) for recovering energy from off-gasses discharged from the scrubber.

Any additional blower (32) may be used for introducing additional air, as may be required.

In order to further retain warmth within the bio cell system, a retractable thermally insulating cover (35), located either inside or outside the tunnel, may be provided for retaining warmth within the bio cell during cooler periods of time such as during night time or wintertime for controlling heat loss.

A controller, generally indicated by numeral (36), has an electronic micro-processor and inputs for connection to the temperature detector (18) and the moisture detector (17); as well as a solar radiation detector (37); any additional sensor (30) that may be present; an external ambient temperature detector (38) and a wireless communications device such as an SMS or other data packet generating unit (39) that is capable of communication with a remote communications device such as a cellular telephone (40) of a person responsible for the operation of the bio cell. In its most desirable format, both communications devices are capable of interacting in both directions so that control settings may be transmitted to the controller from a remote communications device without the person responsible for the operation of the bio cell needing to visit the installation itself. Of course other maintenance may be necessary that requires physical attendance at the bio-cell site.

The controller also has outputs for controlling the circulation pump (12) that controls the flow of heated water from the solar water heater to the heat exchanger according to the temperature detected by the temperature detector (18); the water supply pump (23) for controlling for the flow of water to the sprinklers (21) according to the output from the moisture detector (17); the blower (27) according to the output from the additional sensor (30) that may be an odour sensor; and an automatic position adjustment mechanism (not shown) for automatically adjusting the position of the retractable thermally insulating cover (35).

It is a particular feature of this invention that the entire bio cell installation is self-contained and self energizing and to this end, the controller is energized by an electrical power supply including a battery unit (41) and a solar cell (photovoltaic cell) (42) and associated circuitry for charging the battery unit. The solar cell and battery unit are designed so that they can also energize all the pumps forming part of the system as well as the blower (27) so that the entire bio installation is self energizing. It is of course to be remembered that the heating necessary for warming the bulk material to stimulate microbiological growth is supplied by a renewable energy source, in this instance, by way of the solar water heater (11).

The apparatus of the invention may also include a nutrient detector (45) for detecting nutrients in the leachate or water supplied to the sprinklers in which instance the controller has an input for the output from the nutrient detector and, in the event that the leachate is recycled, a control output to control the addition of nutrients to water/leachate either in the storage tank or in the pipeline as indicated by numeral (46).

It will be understood that, in use, the apparatus described above may be used to conduct a wide range of microbiological processes on bulk materials and that the automatic control of the moisture content, temperature, supply of nutrients, selection of microbiological species and other process variables that target optimum biological activity can be used highly effectively to accelerate microbiological processes especially, but not exclusively, microbiological bioremediation processes.

The controller may be arranged to retain data for a predetermined historic period and to send off appropriate messages via the SMS system to the cellular telephone of a responsible person. The fully stand alone system thus has a communication base station for full technical, physical and biological control. Once the system has been set up for a particular function, it has low operational skill requirements. The system is adaptable for solid or liquid systems, and may even be adaptable for gas phase systems.

On site historical data may be used to direct the bio cell and the various parameters employed. The concentration as well as stability of the contaminant or bio mineral may be monitored in any desired way. The data recovered over a period of time may be used for optimization of the bio cell parameters. If little or no historical data is available the bio cell allows for a simulation of onsite processes before any optimisation occurs and this could give additional information about natural attenuation, plume development and its degradation as well as other variables. Data may be recovered and categorised with respect to topography, microbial phylogeny, geology, geochemistry, climate, etc. These environmental parameters can be used to manage variable conditions of the bio cell.

The bio cell allows for comprehensive analysis including a determination of whether the concentrations of contaminants of concern are stable or decreasing both in time and space.

The system thus allows for the definition of a favorable biochemical and geochemical environment. This means that the redox conditions, oxygen level, concentrations of electron donors and acceptors that are favorable for degradation of the contaminants of concern can be determined, including physicochemical parameters, pH, optimum temperature, water activity, etc. Within a bio cell, the comprehensive microbial diversity and its dynamics may be simulated and evaluated.

Of course establishing a novel tailor made microbial community can increase the rates of degradation to a point where the rate is sufficient or optimized.

The adaptability of the system is a unique feature and therefore extreme environmental conditions and extremophillic reactions are not excluded. It is envisaged that high concentrations of pollutant, high temperatures that will increase the solubility of contaminants, radioactivity, and inert mineral extraction are envisaged as being possible in the system of this invention.

DNA-based tools have been used to monitor microbial diversity in complex communities. Because the environments created by mining, industrial and agricultural activity and the associated waste disposal are so unique, culturing the bacteria is generally extremely challenging. An inability to culture all of the microbes within a complex environment necessitates the use of culture independent methods. There have thus been developed standardized methods and procedures specifically for soil, groundwater and waste samples from impacted environments.

Samples are transported to a laboratory under controlled conditions where microbial diversity assessments may be performed by exponentially increasing targeted areas (PCR amplification) of the genetic starting material (DNA) using probes that target all 3 domains of life (Eukaryotes - nematodes, yeast and fungi, etc. Prokaryotes - bacteria and Archaea). The generated fragments may then be then subjected to a specialized electrophoretic technique that is used to separate these fragments based on compositional differences. Statistical analysis provides a means of comparing and measuring shifts in microbial diversity.

The cost effective, green technology can accelerate catalysis several fold without any additional cost implementation.

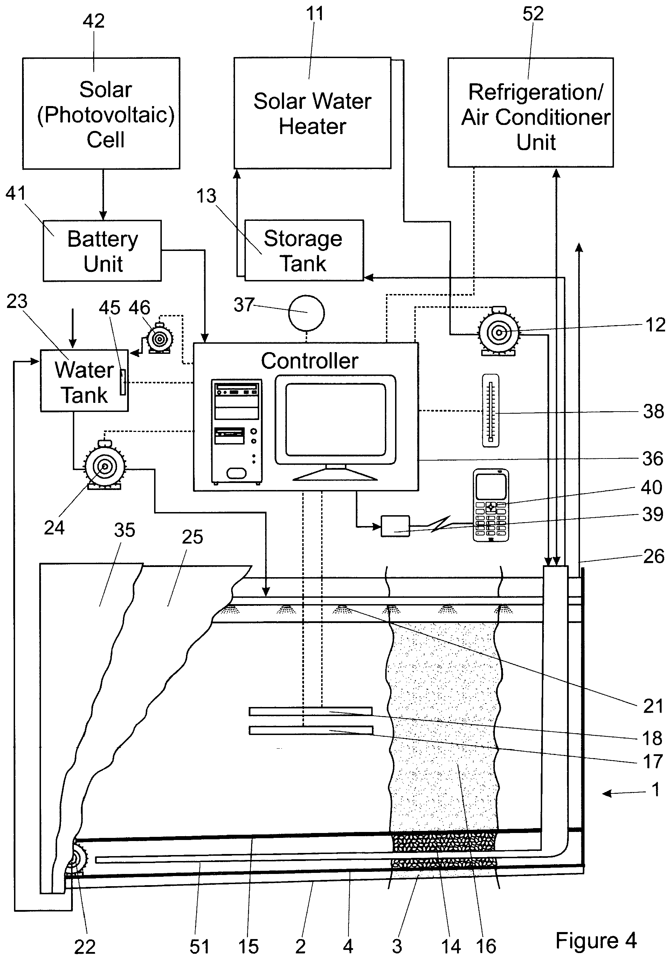

Figure 4 of the drawings illustrates apparatus similar to that described with reference to Figure 1 wherein the same reference numerals are used for the same items of apparatus. The apparatus shown in Figure 4 has, however, the heat exchanger replaced by a simple heat exchanger (51) that transfers heat (or for that matter cold) directly to the permeable body (14) of pebbles or the like for use in instances of anaerobic or anoxic microbes. Of course, the heat exchanger could be buried directly in the body of soil (16) without the permeable body of pebbles should this be appropriate.

Figure 4 also shows a refrigeration or air conditioning unit (52) that can be used for cooling air, in the incidence of aerobic microbes or, water in the instance of anaerobic or anoxic microbes in instances in which ambient temperatures are excessively high and it would be advantageous to cool the body of soil somewhat. It is believed that certain types of energy efficient DC air-conditioners will be appropriate and suitable for the purpose provided that the battery and photovoltaic cells are selected accordingly.

It is thus envisaged that the apparatus could also be used for bacterial metal extraction processes and still further for composting procedures.

## Claims

1. A method of conducting a microbiological process on a bulk material in which a quantity of the bulk material (16) is loaded onto a waterproof lining (4) forming part of a bio cell with a heat transfer arrangement (5) below the quantity of bulk material or within its volume, or both, and wherein the moisture content of the bulk material is controlled by periodic or intermittent distribution of water into the bulk material in order to promote microbiological activity within the bulk material by means of microbes that may be either naturally occurring within the bulk material or may be selected and introduced into the bulk material according to a desired result, and a leachate recovery installation (22, 23) for collecting leachate draining from the bulk material, in use, wherein the temperature within the bulk material is monitored and the heat transfer arrangement is heated or cooled, as may be required, in order to control the temperature thereof to cause the temperature of the bulk material to approach a target temperature associated with enhanced microbial activity of microbes present within the bulk material wherein the heat transfer arrangement operates to heat or cool air that is fed into an air inlet arrangement (5) prior to its discharge into the bulk material, the heat transfer arrangement including a heat exchanger that may be heated or cooled by fluid circulated through the heat exchanger from a suitable source and wherein water is heated in a solar water heater (11) for circulation through the heat exchanger as and when heating of air introduced into the bulk material is required.

2. A method as claimed in claim 1 in which the microbes include aerobic microbes.

3. A method as claimed in either one of claims 1 or 2 in which the microbes include anaerobic microbes.

4. A method as claimed in any one of the preceding claims in which the heat transfer arrangement includes a heat sink composed of a multitude of pebbles or particles (14) having a heat content aimed at maintaining an elevated temperature during periods of time for which the heat source is inactive.

5. A method as claimed in any one of the preceding claims in which nutrients required for a targeted microbial action are added either in the form of solid material at the time that the quantity of bulk material is loaded into the bio cell or by way of water distributed into the bulk material, or both.

6. A method as claimed in any one of the preceding claims in which water is distributed into the bulk material by spraying (21) it on to the upper surface thereof with the water being recycled leachate together with any makeup water that may be added to compensate for losses or to compensate for a bleed stream of leachate that may be removed.

7. A method as claimed in any one of the preceding claims in which the nutrient content of the leachate is monitored and nutrients are added as may be required to leachate that is recirculated.

8. A method as claimed in any one of the preceding claims in which the moisture content of the bulk material is monitored with the distribution of water into the bulk material being controlled according to the moisture content detected.

9. A method as claimed in any one of the preceding claims in which the entire method is carried out in an enclosed environment in the general form of a suitable tunnel (25) in which the tunnel forms an enclosure together with the waterproof lining of the bio cell.

10. Apparatus in the form of a bio cell for the conduct of a method as defined above comprising a waterproof lining (4); a heat transfer arrangement (5) adapted to be covered by a quantity of bulk material (16), in use; a water inlet arrangement (21) including flow regulator means whereby water can be periodically or intermittently distributed in bulk material supported above the waterproof lining; at least one moisture detector (17) for detecting the moisture content of bulk material within the container; a leachate recovery installation (22, 23) for collecting leachate draining from bulk material supported above the waterproof lining in use; and at least one temperature detector (18) for detecting temperature within bulk material supported above the waterproof lining; wherein the heat transfer arrangement is arranged, as may be required in use, to adjust the temperature of bulk material supported above the waterproof lining, wherein the heat transfer arrangement is arranged to heat or cool air that is fed into an air inlet arrangement (5) prior to its discharge into the bulk material, the heat transfer arrangement including a heat exchanger that may be heated or cooled by fluid circulated through the heat exchanger from a suitable source and wherein a solar water heater (11) is included for heating water for circulation through the heat exchanger as and when heating of air introduced into bulk material loaded into the apparatus is required.

11. Apparatus as claimed in claim 10 in which the apparatus includes a controller (38) having an electronic micro-processor with the controller having inputs for association with the at least one temperature detector and the at least one moisture detector; wherein the controller has an output for controlling the flow of heating or cooling fluid to the heat transfer arrangement according to the temperature detected by the at least one temperature detector; the controller also having an output for controlling the flow of water to the water inlet arrangement according to the output from the at least one moisture detector.

12. Apparatus as claimed in claim 11 in which in the microbes to be employed include aerobic microbes and the heat transfer arrangement includes a heat exchanger whereby air fed to an air inlet arrangement is heated according to the temperature of fluid circulated through the heat exchanger from a heat source (11).

13. Apparatus as claimed in either one of claims 11 or 12 in which the controller further has an input for receiving the output from a nutrient detector (45) for detecting nutrients in the leachate and, in the event that the leachate is recycled, the controller has an output for controlling the addition (46) of nutrients to water/leachate being supplied to the water inlet arrangement.

14. Apparatus as claimed in any one of claims 10 to 13 in which the controller has associated with it an electrical power supply including a battery unit (41) and a solar cell arrangement (42) for charging the battery unit and the heat exchanger is connected to a solar water heater assembly (11) to effect heating of the heat exchanger..

15. Apparatus as claimed in any one of claims 10 to 14 in which the heat transfer arrangement is surrounded by a multitude of pebbles or particles (14) having a heat content aimed at maintaining an elevated temperature of inlet air during periods of time for which the heat source is inactive with the pebbles or particles thereby acting as a heat sink.

16. Apparatus as claimed in any one of claims 10 to 15 in which the apparatus includes impervious sheet material forming a tunnel (25) that fully encloses the bio cell with the sheet material and lining of the container together acting to form a total enclosure for the bio cell and wherein an outlet (26) for off-gases is provided in which instance there is provided any appropriate scrubber (28) for removing any harmful components thereof and optionally an auger or turbine for extracting energy from gases leaving the enclosed environment.

17. Apparatus as claimed in any one of claims 10 to 16 in which a retractable insulating cover (35) is associated with the tunnel for selectively controlling heat loss through the tunnel wall according to prevailing external ambient temperature in which instance a controller may be arranged to automatically adjust the position of the retractable insulating cover, according to ambient temperature fed to the controller by an ambient temperature sensor (38).

## Patentansprüche

1. Verfahren zum Durchführen eines mikrobiologischen Prozesses auf einem Schüttgut, bei dem eine Menge des Schüttguts (16) auf eine wasserdichte Auskleidung (4) geladen wird, die einen Teil einer Biozelle bildet, mit einer Wärmeübertragungsanordnung (5) unterhalb der Menge an Schüttgut und/oder innerhalb des Volumens derselben, und wobei der Feuchtigkeitsgehalt des Schüttguts durch eine periodische oder intermittierende Verteilung von Wasser in das Schüttgut hinein gesteuert wird, um eine mikrobiologische Aktivität innerhalb des Schüttguts mittels Mikroben, die entweder natürlicherweise innerhalb des Schüttguts vorkommen können oder gemäß einem gewünschten Ergebnis ausgewählt und in das Schüttgut eingebracht werden können, zu begünstigen, und einer Sickerflüssigkeit-Auffanginstallation (22, 23) zum Sammeln von Sickerflüssigkeit, die bei Verwendung aus dem Schüttgut herausläuft, wobei die Temperatur innerhalb des Schüttguts überwacht wird und die Wärmeübertragungsanordnung nach Bedarf erwärmt oder gekühlt wird, um die Temperatur derselben zu steuern, um zu bewirken, dass die Temperatur des Schüttguts sich einer Solltemperatur nähert, die einer erhöhten Mikrobenaktivität von innerhalb des Schüttguts vorhandenen Mikroben zugeordnet ist, wobei die Wärmeübertragungsanordnung so arbeitet, dass sie Luft, die in eine Lufteinlassanordnung (5) eingeleitet wird, vor ihrer Abgabe in das Schüttgut erwärmt oder kühlt, wobei die Wärmeübertragungsanordnung einen Wärmetauscher enthält, der durch Fluid erwärmt oder gekühlt werden kann, das von einer geeigneten Quelle aus durch den Wärmetauscher zirkuliert, und wobei Wasser zwecks Zirkulation durch den Wärmetauscher in einem Solar-Wassererhitzer (11) erwärmt wird, wenn ein Erwärmen von in das Schüttgut eingebrachter Luft erforderlich ist.

2. Verfahren nach Anspruch 1, bei dem die Mikroben aerobe Mikroben enthalten.

3. Verfahren nach einem der Ansprüche 1 oder 2, bei dem die Mikroben anaerobe Mikroben enthalten.

4. Verfahren nach einem der vorangehenden Ansprüche, bei dem die Wärmeübertragungsanordnung einen Kühlkörper enthält, der aus einer Vielzahl von Kieselsteinen oder Partikeln (14) zusammensetzt ist, die einen Wärmegehalt aufweisen, der auf ein Aufrechterhalten einer erhöhten Temperatur während Zeiträumen, in denen die Wärmequelle inaktiv ist, ausgerichtet ist.

5. Verfahren nach einem der vorangehenden Ansprüche, bei dem Nährstoffe, die für eine gezielte Mikrobenwirkung erforderlich sind, entweder in Form von festem Material zu der Zeit, zu der die Menge an Schüttgut in die Biozelle geladen wird, und/oder mithilfe von Wasser, das in das Schüttgut hinein verteilt wird, zugegeben werden.

6. Verfahren nach einem der vorangehenden Ansprüche, bei dem Wasser in das Schüttgut hinein verteilt wird, indem es auf die obere Oberfläche desselben gesprüht wird (21), wobei das Wasser recycelte Sickerflüssigkeit zusammen mit jeglichem Zusatzwasser, das zugegeben werden kann, um Verluste auszugleichen oder einen möglicherweise entfernten Entnahmestrom von Sickerflüssigkeit auszugleichen, ist.

7. Verfahren nach einem der vorangehenden Ansprüche, bei dem der Nährstoffgehalt der Sickerflüssigkeit überwacht wird und Nährstoffe nach Bedarf der erneut in den Kreis eingebrachten Sickerflüssigkeit zugegeben werden.

8. Verfahren nach einem der vorangehenden Ansprüche, bei dem der Feuchtigkeitsgehalt des Schüttguts überwacht wird, wobei die Verteilung von Wasser in das Schüttgut hinein gemäß dem erfassten Feuchtigkeitsgehalt gesteuert wird.

9. Verfahren nach einem der vorangehenden Ansprüche, bei dem das gesamte Verfahren in einer abgeschlossenen Umgebung in der allgemeinen Form eines geeigneten Tunnels (25) durchgeführt wird, wobei der Tunnel zusammen mit der wasserdichten Auskleidung der Biozelle eine Ummantelung ausbildet.

10. Vorrichtung in Form einer Biozelle zum Durchführen eines Verfahrens, wie vorstehend definiert, Folgendes umfassend: eine wasserdichte Auskleidung (4); eine Wärmeübertragungsanordnung (5), die dazu ausgelegt ist, bei Verwendung durch eine Menge an Schüttgut (16) bedeckt zu sein; eine Wassereinlassanordnung (21), die Durchflussreglermittel enthält, mit denen Wasser periodisch oder intermittierend in oberhalb der wasserdichten Auskleidung getragenes Schüttgut hinein verteilt werden kann; wenigstens einen Feuchtigkeitsdetektor (17) zum Erfassen des Feuchtigkeitsgehalts von Schüttgut innerhalb des Behälters; eine Sickerflüssigkeit-Auffanginstallation (22, 23) zum Sammeln von Sickerflüssigkeit, die bei Verwendung aus oberhalb der wasserdichten Auskleidung getragenem Schüttgut herausläuft; und wenigstens einen Temperaturdetektor (18) zum Erfassen einer Temperatur innerhalb von oberhalb der wasserdichten Auskleidung getragenem Schüttgut; wobei die Wärmeübertragungsanordnung dazu angeordnet ist, je nach Bedarf bei Verwendung die Temperatur von oberhalb der wasserdichten Auskleidung getragenem Schüttgut einzustellen, wobei die Wärmeübertragungsanordnung dazu angeordnet ist, Luft, die in eine Lufteinlassanordnung (5) eingeleitet wird, vor ihrer Abgabe in das Schüttgut zu erwärmen oder zu kühlen, wobei die Wärmeübertragungsanordnung einen Wärmetauscher enthält, der durch Fluid erwärmt oder gekühlt werden kann, das von einer geeigneten Quelle aus durch den Wärmetauscher zirkuliert, und wobei ein Solar-Wassererhitzer (11) zum Erwärmen von Wasser inbegriffen ist, zwecks Zirkulation durch den Wärmetauscher, wenn ein Erwärmen von Luft, die in das in die Vorrichtung geladene Schüttgut eingebracht wird, erforderlich ist.

11. Vorrichtung nach Anspruch 10, bei der die Vorrichtung eine Steuerung (38) enthält, die einen elektronischen Mikroprozessor aufweist, wobei die Steuerung Eingänge für eine Zuordnung zu dem wenigstens einen Temperaturdetektor und dem wenigstens einen Feuchtigkeitsdetektor aufweist; wobei die Steuerung einen Ausgang zum Steuern des Durchflusses von Heiz- oder Kühlfluid zu der Wärmeübertragungsanordnung gemäß der durch den wenigstens einen Temperaturdetektor erfassten Temperatur aufweist; wobei die Steuerung auch einen Ausgang zum Steuern des Durchflusses von Wasser zu der Wassereinlassanordnung gemäß der Ausgabe aus dem wenigstens einen Feuchtigkeitsdetektor aufweist.

12. Vorrichtung nach Anspruch 11, bei der die zu verwendenden Mikroben aerobe Mikroben enthalten und die Wärmeübertragungsanordnung einen Wärmetauscher enthält, wodurch in eine Lufteinlassanordnung eingeleitete Luft gemäß der Temperatur von Fluid, das von einer Wärmequelle (11) aus durch den Wärmetauscher zirkuliert, erwärmt wird.

13. Vorrichtung nach einem der Ansprüche 11 oder 12, bei der die Steuerung ferner einen Eingang zum Empfangen der Ausgabe aus einem Nährstoffdetektor (45) zum Erfassen von Nährstoffen in der Sickerflüssigkeit aufweist und, im Fall, dass die Sickerflüssigkeit recycelt wird, die Steuerung einen Ausgang zum Steuern der Zugabe (46) von Nährstoffen zu der Wassereinlassanordnung zugeführtem/r Wasser/Sickerflüssigkeit aufweist.

14. Vorrichtung nach einem der Ansprüche 10 bis 13, bei welcher der Steuerung eine Stromversorgung zugeordnet ist, die eine Batterieeinheit (41) und eine Solarzellenanordnung (42) zum Aufladen der Batterieeinheit enthält, und der Wärmetauscher mit einer Solar-Wassererhitzerbaugruppe (11) verbunden ist, um ein Erwärmen des Wärmetauschers zu bewirken.

15. Vorrichtung nach einem der Ansprüche 10 bis 14, bei der die Wärmeübertragungsanordnung durch eine Vielzahl von Kieselsteinen oder Partikeln (14) umgeben ist, die einen Wärmegehalt aufweisen, der auf ein Aufrechterhalten einer erhöhten Temperatur von Einlassluft während Zeiträumen ausgerichtet ist, in denen die Wärmequelle inaktiv ist, wobei die Kieselsteine oder Partikel dabei als ein Kühlkörper wirken.

16. Vorrichtung nach einem der Ansprüche 10 bis 15, bei der die Vorrichtung undurchlässiges flächiges Material enthält, das einen Tunnel (25) ausbildet, der die Biozelle vollständig umschließt, wobei das flächige Material und die Auskleidung des Behälters zusammenwirken, um eine vollständige Ummantelung für die Biozelle auszubilden, und wobei ein Auslass (26) für Abgase vorgesehen ist, wobei in diesem Fall ein geeigneter Wäscher (28) zum Entfernen schädlicher Bestandteile derselben und optional eine Schnecke oder Turbine zum Extrahieren von Energie aus die abgeschlossene Umgebung verlassenden Gasen vorgesehen ist.

17. Vorrichtung nach einem der Ansprüche 10 bis 16, bei der dem Tunnel eine zurückziehbare Isolierabdeckung (35) zugeordnet ist, zum gezielten Steuern des Wärmeverlusts durch die Tunnelwand hindurch gemäß der herrschenden äußeren Umgebungstemperatur, wobei in diesem Fall eine Steuerung dazu angeordnet sein kann, die Position der zurückziehbaren Isolierabdeckung gemäß einer der Steuerung durch einen Umgebungstemperatursensor (38) zugeführten Umgebungstemperatur automatisch einzustellen.

## Revendications

1. Procédé de réalisation d'un processus microbiologique sur un matériau en vrac dans lequel une quantité du matériau en vrac (16) est chargée sur un revêtement imperméable (4) faisant partie d'une biopile avec un agencement de transfert de chaleur (5) en dessous de la quantité du matériau en vrac ou à l'intérieur de son volume, ou les deux, et dans lequel la teneur en humidité du matériau en vrac est régulée par une distribution d'eau périodique ou intermittente dans le matériau en vrac afin de favoriser une activité microbiologique à l'intérieur du matériau en vrac au moyen de microbes qui peuvent être naturellement présents à l'intérieur du matériau en vrac ou peuvent être sélectionnés et introduits dans le matériau en vrac selon un résultat souhaité, et une installation de récupération de lixiviat (22, 23) pour recueillir le lixiviat s'écoulant du matériau en vrac, en utilisation, dans lequel la température à l'intérieur du matériau en vrac est surveillée et l'agencement de transfert de chaleur est chauffé ou refroidi, suivant les besoins, afin de réguler sa température pour amener la température du matériau en vrac à s'approcher d'une température cible associée à une activité microbienne améliorée des microbes présents à l'intérieur du matériau en vrac, dans lequel l'agencement de transfert de chaleur fonctionne pour chauffer ou refroidir l'air qui est fourni à un agencement d'orifice d'entrée d'air (5) avant son évacuation dans le matériau en vrac, l'agencement de transfert de chaleur comportant un échangeur de chaleur qui peut être chauffé ou refroidi par un fluide circulant à travers l'échangeur de chaleur à partir d'une source appropriée et dans lequel de l'eau est chauffée dans un chauffe-eau solaire (11) pour une circulation à travers l'échangeur de chaleur si et à chaque fois qu'un chauffage de l'air introduit dans le matériau en vrac est requis.

2. Procédé selon la revendication 1, dans lequel les microbes comportent des microbes aérobies.

3. Procédé selon l'une quelconque des revendications 1 et 2, dans lequel les microbes comportent des microbes anaérobies.

4. Procédé selon l'une quelconque des revendications précédentes, dans lequel l'agencement de transfert de chaleur comporte un dissipateur thermique composé d'une multitude de galets ou de particules (14) ayant une teneur thermique visant à maintenir une température élevée pendant des périodes où la source de chaleur est inactive.

5. Procédé selon l'une quelconque des revendications précédentes, dans lequel des nutriments requis pour une action microbienne ciblée sont ajoutés soit sous forme de matériau solide au moment où la quantité de matériau en vrac est chargée dans la biopile, soit au moyen d'eau distribuée dans le matériau en vrac, ou les deux.

6. Procédé selon l'une quelconque des revendications précédentes, dans lequel de l'eau est distribuée dans le matériau en vrac en la pulvérisant (21) sur la surface supérieure de celui-ci, l'eau étant du lixiviat recyclé conjointement avec toute eau d'appoint qui peut être ajoutée pour compenser des pertes ou pour compenser un courant de purge de lixiviat qui peut être éliminé.

7. Procédé selon l'une quelconque des revendications précédentes, dans lequel la teneur en nutriments du lixiviat est surveillée et des nutriments sont ajoutés lorsque cela est requis au lixiviat qui est remis en circulation.

8. Procédé selon l'une quelconque des revendications précédentes, dans lequel la teneur en humidité du matériau en vrac est surveillée, la distribution d'eau dans le matériau en vrac étant régulée selon la teneur en humidité détectée.

9. Procédé selon l'une quelconque des revendications précédentes, dans lequel la totalité du procédé est réalisée dans un environnement clos sous la forme générale d'un tunnel (25) approprié, dans lequel le tunnel forme une enceinte conjointement au revêtement imperméable de la biopile.

10. Appareil sous la forme d'une biopile pour la réalisation d'un procédé tel que défini ci-dessus comprenant un revêtement imperméable (4) ; un agencement de transfert de chaleur (5) adapté pour être recouvert d'une quantité de matériau en vrac (16), en utilisation ; un agencement d'orifice d'entrée d'eau (21) comportant un moyen régulateur de flux, moyennant quoi de l'eau peut être distribuée de façon périodique ou intermittente dans le matériau en vrac supporté au-dessus du revêtement imperméable ; au moins un détecteur d'humidité (17) pour détecter la teneur en humidité du matériau en vrac à l'intérieur du contenant ; une installation de récupération de lixiviat (22, 23) pour recueillir le lixiviat s'écoulant du matériau en vrac supporté au-dessus du revêtement imperméable en utilisation ; et au moins un détecteur de température (18) pour détecter une température à l'intérieur du matériau en vrac supporté au-dessus du revêtement imperméable ; dans lequel l'agencement de transfert de chaleur est agencé, comme cela est nécessaire en utilisation, pour régler la température du matériau en vrac supporté au-dessus du revêtement imperméable, dans lequel l'agencement de transfert de chaleur est agencé pour chauffer ou refroidir l'air qui est fourni à un agencement d'orifice d'entrée d'air (5) avant son évacuation dans le matériau en vrac, l'agencement de transfert de chaleur comportant un échangeur de chaleur qui peut être chauffé ou refroidi par un fluide circulant à travers l'échangeur de chaleur à partir d'une source appropriée et dans lequel un chauffe-eau solaire (11) est inclus pour chauffer de l'eau pour une circulation à travers l'échangeur de chaleur si et quand un chauffage de l'air introduit dans le matériau en vrac chargé dans l'appareil est requis.

11. Appareil selon la revendication 10, dans lequel l'appareil comporte un dispositif de régulation (38) ayant un microprocesseur électronique, le dispositif de régulation ayant des entrées pour une association avec l'au moins un détecteur de température et l'au moins un détecteur d'humidité ; dans lequel le dispositif de régulation comporte une sortie pour réguler le flux de fluide de chauffage ou de refroidissement vers l'agencement de transfert de chaleur selon la température détectée par l'au moins un détecteur de température ; le dispositif de régulation ayant également une sortie pour réguler le flux d'eau vers l'agencement d'orifice d'entrée d'eau selon la sortie de l'au moins un détecteur d'humidité.

12. Appareil selon la revendication 11, dans lequel les microbes à employer comportent des microbes aérobies et l'agencement de transfert de chaleur comporte un échangeur de chaleur, moyennant quoi de l'air fourni à l'agencement d'orifice d'entrée d'air est chauffé selon la température de fluide circulant à travers l'échangeur de chaleur à partir d'une source de chaleur (11).

13. Appareil selon l'une ou l'autre des revendications 11 et 12, dans lequel le dispositif de régulation comporte en outre une entrée pour recevoir la sortie d'un détecteur de nutriment (45) pour détecter des nutriments dans le lixiviat et, en cas d'un recyclage du lixiviat, le dispositif de régulation comporte une sortie pour réguler l'addition (46) de nutriments à l'eau/au lixiviat qui est fourni à l'agencement d'orifice d'entrée d'eau.

14. Appareil selon l'une quelconque des revendications 10 à 13, dans lequel le dispositif de régulation est associé à une alimentation électrique comportant une unité de batterie (41) et un agencement de pile solaire (42) pour charger l'unité de batterie et l'échangeur de chaleur est raccordé à un ensemble chauffe-eau solaire (11) pour effectuer le chauffage de l'échangeur de chaleur.

15. Appareil selon l'une quelconque des revendications 10 à 14, dans lequel l'agencement de transfert de chaleur est entouré d'une multitude de galets ou de particules (14) ayant une teneur thermique visant à maintenir une température élevée de l'air entrant pendant des périodes où la source de chaleur est inactive, les galets ou les particules agissant ainsi comme un dissipateur thermique.

16. Appareil selon l'une quelconque des revendications 10 à 15, dans lequel l'appareil comporte un matériau de feuille étanche formant un tunnel (25) qui enferme complètement la biopile, le matériau de feuille et le revêtement du contenant agissant ensemble pour former une enceinte totale pour la biopile et dans lequel un orifice de sortie (26) pour des dégagements gazeux est prévu, auquel cas il est prévu tout épurateur (28) approprié pour en éliminer tout composant nocif et facultativement une tarière ou une turbine pour extraire l'énergie des gaz quittant l'environnement clos.

17. Appareil selon l'une quelconque des revendications 10 à 16, dans lequel un couvercle isolant rétractable (35) est associé au tunnel pour réguler sélectivement une perte de chaleur à travers la paroi de tunnel selon une température ambiante externe prédominante, auquel cas un dispositif de régulation peut être agencé pour régler automatiquement la position du couvercle isolant rétractable, selon la température ambiante fournie au dispositif de régulation par un capteur de température ambiante (38).
